# EUROPEAN PATENT APPLICATION

(11) **EP 2 002 856 A1**
(43) Date of publication of application: **17.12.2008**
(21) Application number: 07388044.5
(22) Date of filing: 15.06.2007
(51) Int. Cl.: A61M 15/06, B65D 25/08

(54) **Multiple substance compartment duct container**

(71) Applicant: TrendTech A/S, 8620 Kjellerup (DK)
(72) Inventor: Jensen, Anders Leonhard, 8620 Kjellerup (DK); Debess, Tage Wenzel, 3150 Hellebæk (DK)
(74) Representative: Boesen, Johnny Peder

(57) **Abstract**

The present invention relates to a duct container for an inhalator and for storing substances to be mixed with other substances, said duct container comprises at least two elastic dividers elastically restrained in said duct and forming at least one compartment there between and wherein said at least one substance is restrained, said elastic dividers being movable in said duct such that said at least one substance restrained in said compartment can be released from said compartment and mixed with other substances. The invention further relates to a method of manufacturing the duct container and an inhalator comprising the duct container.

## Description

The present invention relates to an air tight duct container allowing two or more substances to be packed such that they do not interact with each other and easily can be mixed. The invention further relates to an inhalator comprising such duct container and where the substances to be inhaled are stored in the duct container.

### BACKGROUND

Often a need to store two or more reactive substances together but separated, e.g. two component glue, penicillin and water or interactive substances, is seen. When mixed, such substances will often initiate a reaction.

Further, reactive substances when mixed can degrade each other by oxidation, reduction or by forming combination products. Likewise, the presence of air can sometimes interact with substances in an unwanted way. In connection with inhalers where two or more substances need to be mixed and inhaled it is often necessary to store the substances separately prior to inhalation.

### OBJECT AND SUMMARY OF THE INVENTION

It is therefore an object of the present invention to provide a storageable end product that allows for longer shelf life, which overcomes some or all of the above mentioned problems.

This is achieved by a duct container for an inhalator and for storing substances to be mixed with each other, said duct container comprising at least two elastic dividers elastically restrained in said duct and forming at least one compartment there between, and wherein said at least one substance is restrained, said elastic dividers being movable in said duct such that said at least one substance restrained in said compartment can be released from said compartment and mixed with other substances.

Hereby it is possible to store different substances in separate compartments and easily mix the different substances, because the dividers between the compartments can easily by removed/broken. The elastic dividers can be positioned such that a multiple number of compartments will be created between the elastic dividers. The elastic dividers will due to their elasticity be restrained inside the duct and can easily be removed by providing a force to the elastic dividers, such that they will be pushed or pulled out of the duct and thereby allow the substance to be removed/released from the compartment. The substances can thereafter easily be mixed e.g. in another container or a mixing compartment. The elastic dividers could in the case of fluid substances be adapted to elastically expand towards the entire wall of the duct and thereby create a tight sealing between the compartments preventing the fluid substances from diffusing between the compartments and also preventing air from entering the compartment. The elastic dividers could be formed by any elastic material such as rubber, polymers, silicone, or comprise mechanic, elastic parts and would be constructed such that they would be squeezed together prior to being inserted into the duct. The elastic means would hereafter expand towards their equilibrium state and thus be elastically restrained inside the duct if the elastic dividers in their equilibrium state are larger than the cross section of the duct. The duct could be any kind of duct forming a cavity, and through which substances can flow, and can have any geometric shape e.g. ducts with cylindrical, rectangular, and/or variable cross sections, curved and/or straight ducts etc.

In another embodiment the elastic dividers are formed as spheres. Hereby slightly oversized silicone spheres or balls e.g. formed of rubber, plastic or silicone could be used as elastic dividers thereby creating a diffusion tight seal between the compartments - and thereby preventing fluid substance from diffusing out of the compartment and preventing air from entering the compartment.

In another embodiment at least a part of the surface of said elastic dividers is surface treated enhancing liquid adherence properties of said surface, such that said substance will adhere to said surface of said elastic dividers. Hereby is achieved that fluid substances can easily evaporate when released from a compartment, because fluid substances would adhere to the surface of the elastic dividers e.g. when pushed/pulled into a mixing compartment and due to a larger surface area of the elastic dividers. The surface of said elastic dividers might be surface treated in order to enhance liquid adherence properties of the surface in a number of different ways. The elastic dividers could for instance be produced of silicone or rubber having ancillary substances which would help liquid substances adhere to the surface. The elastic dividers could also be mechanically formed such that liquids would adhere to the surface - for instance such that the surface is granulated and/or rough. The surface treatment could also be a chemical treatment where a chemical substance has been provided onto the surface of the elastic dividers, and the chemical substance could then react with liquid substances and thereby improve the adherence properties.

The present invention further discloses an inhalator comprising a duct container as described above where at least one of said substances is inhaled through said duct. Hereby it is possible to construct an inhalator comprising one or more compartments with substances to be inhaled and the user can - simply by pushing/pulling the elastic dividers - release the substances to be inhaled. In connection with inhalators for inhaling nicotine and or aroma substances the present invention provides safe storage of nicotine and aroma substances in a separated fashion so they will not interact prematurely and are shielded from air whereby longer shelf life is ensured. Such inhalator could for instance be activated by blowing the elastic dividers out of the duct with the mouth or by using a simple exterior pin.

In another embodiment the inhalator further comprises a mixing compartment having a larger cross section than the cross section of said duct, said mixing compartment being placed in extension of said duct, such that said elastic dividers can be moved into said mixing compartment allowing said substances to mix. The substances and elastic dividers could e.g. be pushed into a mixing compartment where the substances could be mixed and evaporate allowing the user to inhale the evaporated substances through the duct. In another embodiment the mixing compartment comprises at least a second substance to be mixed with said at least one substance restrained in said duct container. Hereby it is achieved that only one of the substances to be mixed needs to be pushed out of the duct container and into the mixing compartment.

In another embodiment the inhaler comprises at least one partially air permeable barrier preventing said elastic dividers from exiting said inhalator. The permeable barriers could for instance be integrated in said mixing compartment and/or in a part of said duct. Hereby it is possible to provide a barrier preventing the elastic dividers from moving out of the mixing compartment and/or be inhaled by the user through the duct and at the same time allow other substances, e.g. air, to flow through the mixing compartment. The permeable divider could be formed by any material being at least air permeable such as membranes, filter material or could be embodied as flanges inside the inhalator preventing the elastic dividers from escaping.

The present invention further relates to a manufacturing method for manufacturing a duct container as described above, said manufacturing method comprises the steps of:
- positioning a first elastic divider in said duct by pushing said elastic divider into said duct;
- dosing a least one substance into said duct beside said first elastic divider;
- positioning a second elastic divider in said duct beside said substance while compressing said second elastic divider such that airflow between said duct and said second elastic divider is at least partially allowed.

Hereby a fast, cheap, and simple method for manufacturing a duct container according to the present invention is provided. The second elastic divider is compressed while positioning it inside the duct, and airflow between the duct and the feeding probe is therefore allowed. Hereby is avoided that high air pressure is generated inside the compartments between the elastic dividers.

In another embodiment the manufacturing method further comprises the steps of:
- repeating a number of times the steps of dosing at least one substance into said duct beside a first elastic divider and positioning a second elastic divider in said duct beside said substance while compressing said elastic divider such that airflow between said duct and said second elastic divider is at least partially allowed.

Hereby a multiple number of substances can be stored separately in the compartments.

In another embodiment of the manufacturing method the step of positioning a first elastic divider comprises the steps of:
- inserting a feeding probe into said duct, said feeding probe having a smaller cross sectional area than said duct and comprising a number of elastic dividers inserted into said feeding probe;
- positioning a first elastic divider into said duct by pushing anelastic divider out of said feeding probe;
- removing said feeding probe from said duct;
and where said step of dosing a least one substance into said duct beside said first elastic divider comprises the step of:
- dosing said substance into said duct beside said first elastic divider, by inserting a dosing probe into said duct;
and where said step of positioning a second elastic divider in said duct comprises the steps of:
- inserting said feeding probe by pushing a second elastic divider out of said feeding probe.

The elastic dividers could hereby be inserted in a feeding probe having a slightly smaller cross section than the duct and could therefore easily be inserted into the duct. An elastic divider could then be pushed out of the feeding probe by pushing the elastic dividers inside the feeding probe, hereby positioning the elastic divider and elastically restraining it inside the duct. The feeding probe could thereafter easily be removed and substances could be dosed into the duct, whereafter a second elastic divider is positioned inside the duct. The different steps of the manufacturing method could be performed simultaneously - e.g. by implementing the manufacturing method in a conveyor production system.

Summarizing, the present invention provides the following advantages:

### Advantages in general:

- low production price
- separation of substances
- long durability/storage life
- alternative dose dispensing device for composite medicals

### Advantages from the duct and spheres:

- in the production: fast, inexpensive, easy to change the production into other sizes or more compartments,
- longer storage life (as the substances do not mix or get exposed to air)
- child-resistant and breakage-proof
- user-friendly, easy-to-use
- could be designed for smoke cessation
- shape of compartments and use of compressible spheres offer possibility for substances to expand without leakage.

### Advantages from the production of the compartments:

- dynamic separation of chambers
- two or more compartments per production cycle
- fast, simple yet inexpensive

### BRIEF DESCRIPTION OF THE DRAWINGS

In the following, preferred embodiments of the invention will be described referring to the figures, where
Figure 1a - 1b illustrate an embodiment of an inhalator with a duct container according to the invention, where figure 1 a and 1 b illustrate how the compartments can be activated by a pin and by an air blow, respectively.
Figure 2 illustrates another embodiment of an inhalator with a duct container according to the invention,
Figure 3a - 3c illustrate other embodiments of an inhalator with a duct container embodied as a separate unit.
Figure 4 illustrates an enlarged part of the duct container of figure 3, and further illustrates the mouth piece end of the duct container.
Figure 5 illustrates an enlarged part of the inhalator of figure 3, and further illustrates a front view of an air permeable barrier.
Figure 6a - 6c illustrate other embodiments of an inhalator with a duct container embodied as a separate unit having a conic shape.
Figure 7 illustrates a method of fabricating a duct container, including placement of spheres and dosing according to the invention.

### DETAILED DESCRIPTION OF EMBODIMENTS

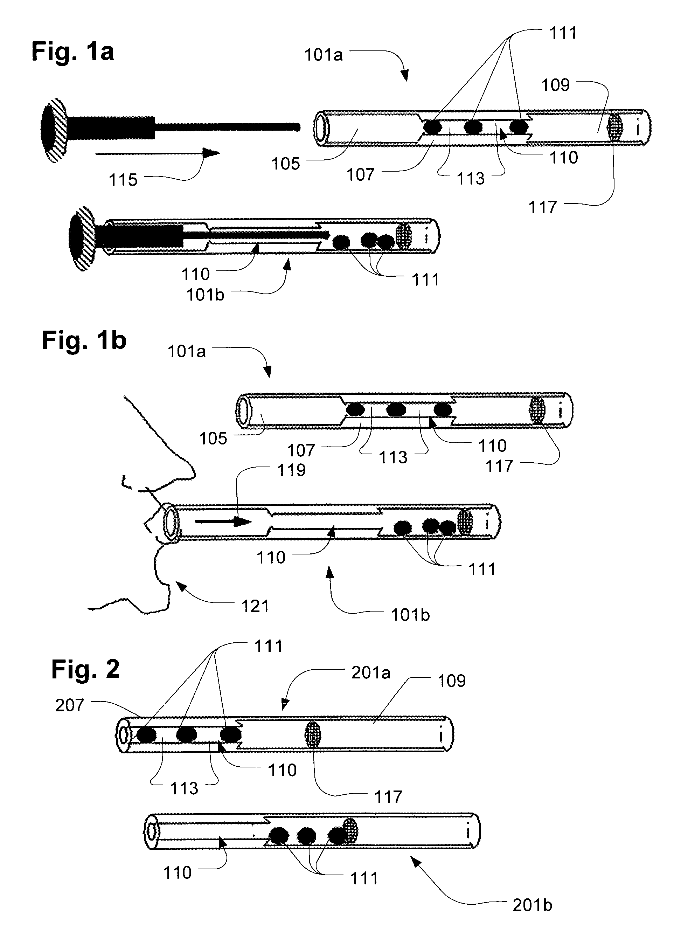

The duct container according to the present invention is in the following illustrated in connection with an inhalator but could also be used in connection with other applications where two or more substances need to be stored separately. Figure 1-6 illustrate embodiments of a duct container integrated in an inhalator, and the different parts of the container/inhaler according to the present invention are shown in different situations and views

In figure 1 a is shown a cross sectional view of an inhalator prior to activation (101a) as well as during activation (101b) via a pin (103). The inhaler is formed as a hollow cylinder comprising a mouth piece (105), a duct container (107) and an end piece (109). The mouth piece and the end piece are formed as a thin walled duct, and a thickening of the walls defines the duct container which has a duct (110) with smaller diameter compared to the mouth piece and end piece, and a user can inhale air through the duct. The duct container (107) comprises a number of elastic spheres (111) that is elastically restrained in the duct (110). Prior to use, the substance or substances, which are to be released and inhaled by the user, are kept in the compartments (113) defined by the space between two or more spheres (111). The inhaler can be activated by inserting a pin (103) into the duct container as illustrated by arrow (115), and the pin will push the elastic spheres into the end piece. Substances kept in different compartments are thus allowed to mix and blend and are exposed to air whereby the different drugs evaporate and are made available for the user to inhale. The pin is then removed whereby a free passage of air is provided, and the user could inhale the substances through the mouth piece. These spheres (111) are made of for instance compressible silicone, rubber, neoprene or the like. In some applications, the use of a special skin forming, cell foamed silicone can be advantageous. The spheres could also have a granulated (not shown) surface whereby the substances would have a larger area to evaporate from when the inhalator is activated. Figure 1b illustrates another way of activating the inhalator. Here the inhalator is activated by a user (121) blowing (119) air into the duct and hereby forcing the spheres out in the end piece of the inhalator. Using this method a pin will not be needed. Either way, the spheres will act to wipe off and push all available substance to the end piece and thereby ensuring that the substances mix.

One or two air permeable barriers or filters are placed in the inhaler. The filter (117) in one end ensures that none of the substances or spheres can possible exit the inhalator duct, even if the liquids have not fully evaporated. The filter can for instance be an acetate tow filter, a cellulose filter or a sintered polypropylene filter. A similar filter placed in the mouth piece can reduce the risk of liquids entering the mouth of the user (not shown).

Figure 2 illustrates another embodiment of the inhalator prior to activation (201 a) and after activation (201 b). The duct container (207) is in this embodiment placed in one end of the inhalator and constitutes thus a part of the mouth piece.

If the inhaler is to be used as a smoke-free cigarette, i.e. for inhaling nicotine, it is advantageous to keep the nicotine substance in the closed compartments (113) without exposure to the air, whereby the durability of the product will be significantly longer. Further, if any aromatic substance or substances or helping agents are to be inhaled in combination with the nicotine, these aromatic substances are advantageously kept in one or more compartments (113) separate from the nicotine substance. Such aromatic substances could be of the group of Beta damascenone, 4-oxo-beta-ionone, oxo-edulan I-II, 3-oxo-alpha-ionone, Dihydroactionodiolide, Megastigmatrienones (4 isomer), different Carotinoide derivate and the helping agents could for instance be different air humidifying agents, pH regulating fluids (such as picrin acid or ammonia), propylene glycol, catalysts, emulsifiers, Tannin (astringent) or different naturopathic drugs.

The different parts and modules of the inhaler can be made of different plastic materials, Barex, metal alloys, or silicone To give the user a more realistic feeling of holding and 'smoking' a cigarette, the inhaler is in one embodiment wrapped with cigarette and filter paper.

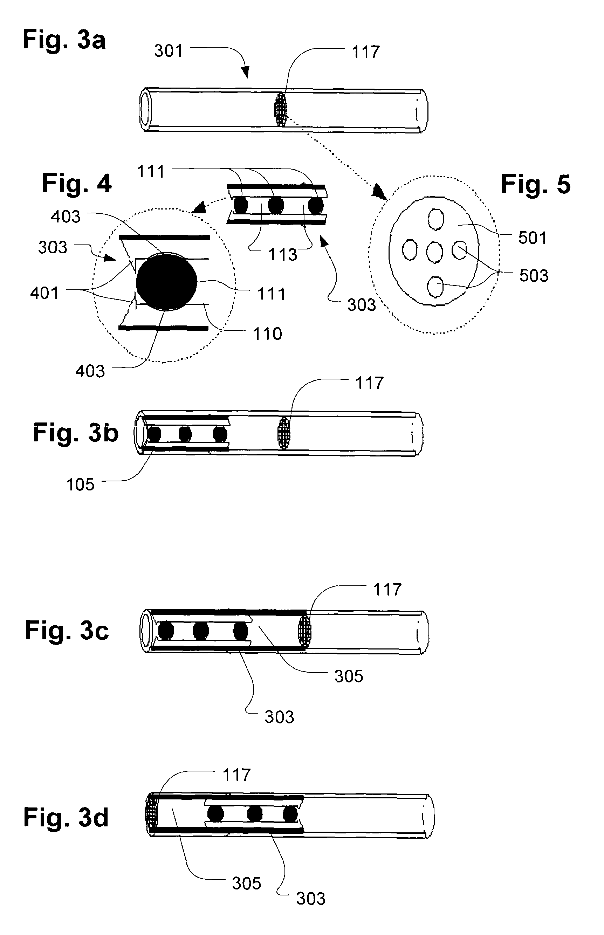

Figure 3a - 3d illustrate other embodiments of an inhalator with a duct container, where figure 3a illustrates that the inhalator comprises a primary duct (301) and a separate duct container (303) both embodied as a separate unit. Figure 3b, 3c and 3d illustrate different embodiments of the primary duct and the separate duct container brought together. The primary duct could for instance be an extruded tube, and the separate duct container (303) comprises a number of elastic spheres (111) forming sealed compartments (113) with the substances to be mixed and inhaled as described above. Once the separate duct container has been produced, it can be inserted into the primary duct as illustrated in figure 3b, 3c, and 3d. Figure 3b illustrates that the separate duct container (303) has been inserted in one end of the primary duct (301) and this end forms the mouth piece of the inhalator (105). The primary duct further comprises an air permeable barrier (117) placed a distance from the separate duct unit, such that a mixing and evaporation chamber (305) are formed within the primary duct. The inhalator could then be activated by pushing or blowing the spheres into the evaporation chamber as described above. Figure 3c and 3d illustrate an embodiment where the air permeable barrier and mixing and evaporation chamber has been integrated into the separate duct container and thus can be produced separately and thereafter inserted into the primary duct (301). Figure 3c and 3d illustrate that the separate duct container can be inserted into the primary tube in different ways; however, a person skilled in the art would be able to construct a number of embodiments differing from those illustrated.

Figure 4 illustrates an enlarged part of the mouth piece end of the duct container (303). The duct container comprises in this embodiment an air permeable barrier formed as a flange/shoulder (401) in the duct wall. The flange/shoulder forms a narrowing in the duct. The narrowing is constructed so small that the elastic spheres cannot be squeezed through the narrowing. Hereby the spheres are prevented from being pushed out through the mouth piece of the inhaler. Figure 4 further illustrates that the duct wall comprises a restraining groove (403) into which a part of the elastic sphere is elastically restrained. The restraining groove has in one embodiment the same curvature as the elastic sphere which creates a tight sealing between the sphere and the duct wall.

Figure 5 illustrates an enlarged view of an air permeable barrier seen from the front. The barrier is formed as a disc (501) that fits into the primary duct. The disc comprises a number of cuttings allowing air to pass through the barrier, and the cuttings are so small that the spheres cannot be pushed through the disc. The disc could in one embodiment also be molded into the primary duct.

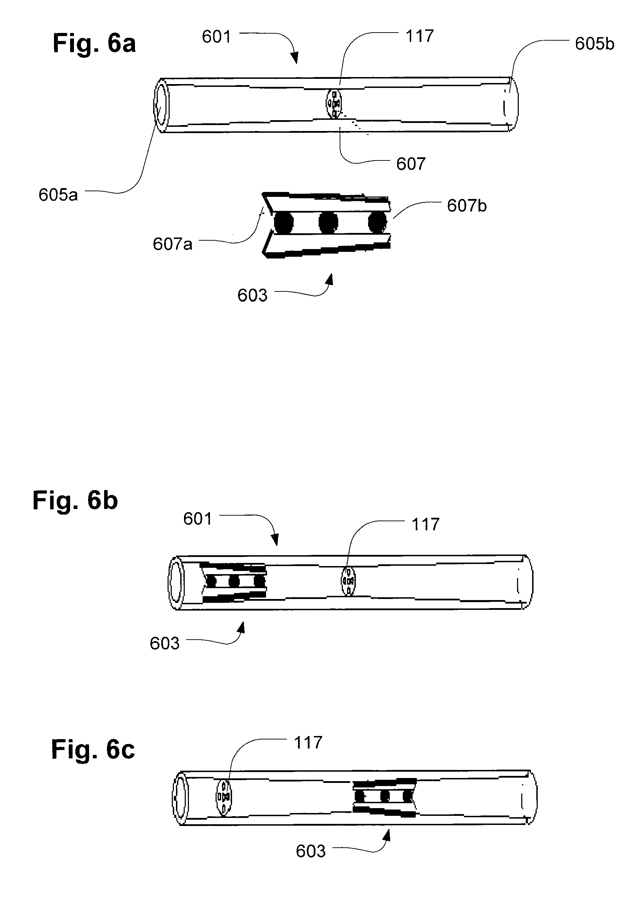

Figure 6a - 6c illustrate other embodiments of the inhalator with a duct container, where figure 6a illustrates that the inhalator comprises a primary duct (601) and a separate duct container (603) embodied as separate units, and figure 6b and 6c illustrate different embodiments of the of the primary duct and the separate duct container brought together. The primary duct is in this embodiment formed as a conic duct which narrows from the ends (605a, 605b) towards the center (607) of the duct, and the outer surface of the separate duct container has been formed with substantially the same conic shape as the primary duct. The separate duct container is thus wider in one end (607a) than in the other end (607b). The consequence is that the separate duct container can be inserted into the conic primary duct (610) and can be squeezed and secured inside the conic duct as illustrated in figure 6b and 6c. Hereby the primary duct and the separate duct container could be constructed separately, and the separate duct container can easily be inserted into the primary duct container.

The modular construction of the inhaler is also advantageous for recycling purposes in that the used and disposed inhaler can easily and fast be broken apart and separated into its module parts and sorted accordingly. In this way, any metals and/or plastics used in some of the modules can easily be sorted out for recycling.

The duct (110) of the duct container could in one embodiment further comprise a helical rifle groove (not shown) forcing the spheres to rotate when they are being pushed out of the duct. This would cause liquid substances stored in the compartments (113) to be greased onto the entire surface area of the spheres, and the liquid substances would thereby easier evaporate when the spheres and substances have been pushed out of the duct (110).

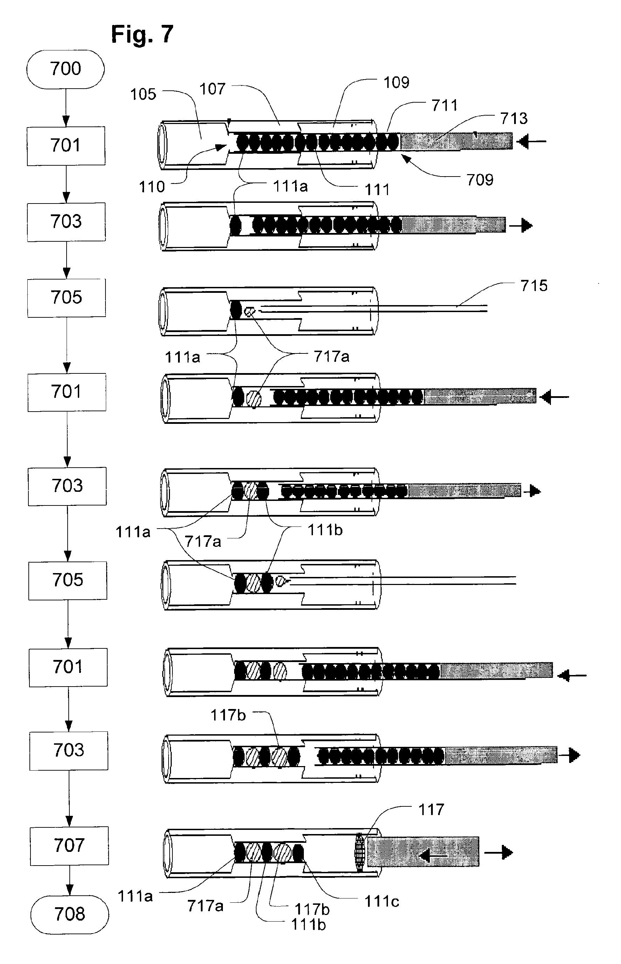

Figure 7 illustrates a method of manufacturing an inhalator comprising a duct container according to the present invention. An inhalator (101) as described in figure 1 and without the elastic spheres and substances is provided (700) prior to manufacturing. Such an inhalator could for instance be produced using known molding techniques.

In step (701) a filling probe (707) having a slightly smaller diameter than the duct (110) of the duct container (107) is inserted into the duct of the duct container. The filling probe is formed as a pipe (711) and comprises a bulk of compressed spheres (111). A first sphere is pushed out of the filling probe by a pushing tool (713) (e.g. a mechanical piston or by using air pressure). The first elastic sphere (111a) is then released from the filling probe and would expand end be elastically restrained inside the duct (110). The filing probe is in step (703) pulled out of the duct, and a dosing probe (715) is in step (705) inserted into the duct. The dosing probe could be a pipe through which substances (717a) could be dosed into the duct, and the dosing probe would be retracted from the duct when a first substance has been dosed into the duct. Steps (701) and (703) are hereafter repeated in order to insert a second elastic sphere (111 b) into the duct, whereby the first substance is kept in an air tight compartment. The second elastic sphere is compressed inside the feeding probe and airflow between the duct and the feeding probe is allowed since the feeding probe has a slightly smaller diameter than the duct. Hereby the generation of a high air pressure inside the compartment (113) is avoided. Further substances (117b) could be inserted by repeating step (705), (701) and (703) a number of times. In step (707) an air permeable barrier 8117) as described above is inserted into the inhaler, e.g. by groove and ridge fit, or by gluing or welding, to ensure against breakage.

Alternatively to compressing the second sphere while positioning it inside the duct by using the feeding probe, a small tube could be placed inside and along the duct wall. The second sphere could then be pushed into the duct, and air could flow out of the compartment (113) through the small tube. The small tube could then be pulled out of the duct, once the second sphere has been correctly positioned, and the sphere would expand and create a tight sealing.

In step (708) an inhalator comprising multiple compartments is provided. Due to the design, the production of the inhaler becomes very fast, simple and cost-effective. Furthermore, the production can very easily be adjusted to specific demands of different lengths/volumes, different combinations of substances used, different types of mouth pieces etc. It is also straightforward and fast to change the production into making tubes with more initially separated compartments of substances by simply adding one or more extra compartment dividing sphere.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word 'comprising' does not exclude the presence of other elements or steps than those listed in a claim.

## Claims

1. A duct container e.g. for an inhalator and for storing substances to be mixed with each other, said duct container comprises at least two elastic dividers elastically restrained in said duct and forming at least one compartment there between, and wherein said at least one substance is restrained, said elastic dividers being movable in said duct such that said at least one substance restrained in said compartment can be released from said compartment and mixed with other substances.

2. A duct container according to claim 1 where said elastic dividers are formed as spheres.

3. A duct container according to claims 1-2, where at least a part of the surface of said elastic dividers are surface treated enhancing liquid adherence properties of said surface, such that said substance would more easily adhere to said surface of said elastic dividers.

4. An inhalator comprising a duct container according to claims 1-3 where at least one of said substances is inhaled through said duct.

5. An inhalator according to claim 4, where said inhalator further comprises a mixing compartment having a larger cross sectional area than the cross sectional area of said duct, said mixing compartment being placed in extension of said duct, such that said elastic dividers can be moved into said mixing compartment allowing said substances to mix .

6. An inhalator according to claim 5, where said mixing compartment comprises a least a second substance to be mixed with said at least one substance restrained in said duct container.

7. An inhalator according to claims 4-6, where said inhalator further comprises at least one partially air permeable barrier preventing said elastic dividers from exiting said inhalator.

8. A manufacturing method for manufacturing a duct container according to claims 1-3, said manufacturing method comprises the steps of:
- positioning a first elastic divider in said duct by pushing said elastic divider into said duct;
- dosing a least one substance into said duct beside said first elastic divider;
- positioning a second elastic divider in said duct beside said substance while compressing said second elastic divider such that airflow between said duct and said second elastic divider is at least partially allowed.

9. A manufacturing method according to claim 8, said manufacturing method further comprises the steps of:
- repeating a number of times the steps of dosing a least one substance into said duct beside a first elastic divider and positioning a second elastic divider in said duct beside said substance while compressing said elastic divider such that airflow between said duct and said second elastic divider is at least partially allowed.

10. A manufacturing method according to claims 8-9, where said step of positioning a first elastic divider comprises the steps of:
- inserting a feeding probe into said duct, said feeding probe having a smaller cross sectional area than said duct and comprises a number of elastic dividers inserted into said feeding probe;
- positioning a first elastic divider into said duct by pushing an elastic divider out of said feeding probe;
- removing said feeding probe from said duct;
and where said step of dosing a least one substance into said duct beside said first elastic divider comprises the step of:
- dosing said substance into said duct beside said first elastic divider, by inserting a dosing probe into said duct;
and where said step of positioning a second elastic divider in said duct comprises the steps of:
- inserting said feeding probe and by pushing a second elastic divider out of said feeding probe.
